# EUROPEAN PATENT SPECIFICATION

(11) **EP 1 896 380 B1**
(45) Date of publication and mention of the grant of the patent: **29.02.2012**
(21) Application number: 06744810.0
(22) Date of filing: 05.06.2006
(51) Int. Cl.: C07B 59/00, C07C 67/36

(54) **METHOD FOR THE USE OF [11C]CARBON MONOXIDE IN LABELING SYNTHESIS OF 11C-LABELLED ESTERS BY SENSITIZED PHOTO-INDUCED FREE RADICAL CARBONYLATION**
VERFAHREN ZUR VERWENDUNG VON [11C]KOHLENMONOXID ZUR MARKIERENDEN SYNTHESE VON 11C-MARKIERTEN ESTERN DURCH SENSIBILISIERTE PHOTOINDUZIERTE RADIKALISCHE CARBONYLIERUNG
PROCÉDÉ D'UTILISATION DE MONOXYDE DE CARBONE [11C]DANS LE MARQUAGE DE LA SYNTHÈSE D'ESTERS MARQUÉS 11C-PAR CARBONYLATION RADICALAIRE SENSIBILISÉE PHOTOINDUITE

(30) Priority: 08.06.2005 US 688469 P
(43) Date of publication of application: 12.03.2008
(73) Proprietor: GE Healthcare Limited, Little Chalfont Buckinghamshire HP7 9NA (GB)
(72) Inventor: LANGSTROM, Bengt, S-751 09 Uppsala (SE); ITSENKO, Oleksiy Department of Organic Chemistry, Box 599, Bmc S-751 24 Uppsala (SE)
(74) Representative: Canning, Lewis R.
(86) International application number: PCT/IB2006/001452
(87) International publication number: WO 2006/131803

(56) References cited:
- WO-A-02/102711
- WO-A-2005/042441
- ITSENKO, O. AND LANGSTRÖM, B.: "Photoinitiated Free Radical Carbonylation Enhaced by Photosensitizers" ORGANIC LETTERS, vol. 7, no. 21, 2005, pages 4661-4664, XP002385444
- ITSENKO ET AL: "Synthesis of aliphatic [carbonyl-11C] esters using [11C]carbon monoxide" EUROPEAN JOURNAL OF ORGANIC CHEMISTRY, WILEY-VCH VERLAG, WEINHEIM, DE, no. 17, 2005, pages 3830-3834, XP002385446 ISSN: 1434-193X
- KIHLBERG T ET AL: "[11C]CARBON MONOXIDE IN SYNTHESIS OF 11C-LABELLED ESTERS USING PALLADIUM-MEDIATED REACTIONS WITH ORGANO HALIDES" JOURNAL OF LABELLED COMPOUNDS AND RADIOPHARMACEUTICALS, JOHN WILEY, CHICHESTER, GB, vol. 44, no. SUPPL 1, 2001, pages S990-S992, XP008063022 ISSN: 0362-4803
- THORELL J-O ET AL: "PREPARATION OF [11C]DIETHYL OXALATE AND [11C]OXALIC ACID AND DEMONSTRATION OF THEIR USE IN THE SYNTHESIS OF [11C]-2,3-DIHYDROXYQUINOXALINE" JOURNAL OF LABELLED COMPOUNDS AND RADIOPHARMACEUTICALS, JOHN WILEY, CHICHESTER, GB, vol. 33, no. 11, 1993, pages 995-1005, XP008063021 ISSN: 0362-4803
- WINSTEAD M B ET AL: "Relationship of molecular structure to in vivo scintigraphic distribution of carbon-11 labeled compounds. 4. carbon-11 labeled mandelonitriles, mandelic acids and their esters" JOURNAL OF MEDICINAL CHEMISTRY, AMERICAN CHEMICAL SOCIETY. WASHINGTON, US, vol. 21, no. 2, 1978, pages 215-217, XP002377121 ISSN: 0022-2623
- KIYOTO NAGAHARA ET AL: "Radical Carboxylation: Ester Synthesis from Alkyl Iodides, Carbon Monoxide, and Alcohols under Irradiation Conditions" JOURNAL OF THE AMERICAN CHEMICAL SOCIETY, AMERICAN CHEMICAL SOCIETY, WASHINGTON, DC, US, vol. 119, 1997, pages 5465-5466, XP002377120 ISSN: 0002-7863
- ITSENKO ET AL: "Radical-mediated carboxylation of alkyl iodides with [11C]carbon monoxide in solvent mixtures" JOURNAL OF ORGANIC CHEMISTRY, AMERICAN CHEMICAL SOCIETY. EASTON, US, vol. 70, no. 6, 2005, pages 2244-2249, XP002385443 ISSN: 0022-3263
- ALLEN R.E.: 'The Concise Oxford Dictionary of Current English', 1992, OXFORD UNIVERSITY PRESS, OXFORD page 964

## Description

### Field of the Invention

The present invention relates to a method for the use of carbon-isotope monoxide in labeling synthesis. More specifically, the invention relates to a method for producing an [¹¹C]carbon monoxide enriched gas mixture from an initial [¹¹C]carbon dioxide gas mixture, and using the produced gas mixture in labeling synthesis by photo-initiated carbonylation. Radiolabeled esters are provided using alcohols, respectively, and alkyl or aryl iodides as precursors, as well as photosensitizes

### Background of the Invention

Tracers labeled with short-lived positron emitting radionuclides (e.g. ¹¹C, t_{1/2} = 20.3 min) are frequently used in various non-invasive in vivo studies in combination with positron emission tomography (PET). Because of the radioactivity, the short half-lives and the submicromolar amounts of the labeled substances, extraordinary synthetic procedures are required for the production of these tracers (see for example Thorell et al., Label Comp. Radiopharm, 1993, 995-1005; Winstead et al., J. Med. Chem. 1978, 215-217, describing the synthesis of "C labelled acids). An important part of the elaboration of these procedures is development and handling of new ¹¹C-labelled precursors. This is important not only for labeling new types of compounds, but also for increasing the possibility of labeling a given compound in different positions.

During the last two decades carbonylation chemistry using carbon monoxide has developed significantly. The recent development of methods such as palladium-catalyzed carbonylative coupling reactions has provided a mild and efficient tool for the transformation of carbon monoxide into different carbonyl compounds.

Carbonylation reactions using [¹¹C]carbon monoxide has a primary value for PET-tracer synthesis since biologically active substances often contain carbonyl groups or functionalities that can be derived from a carbonyl group. The syntheses are tolerant to most functional groups, which means that complex building blocks can be assembled in the carbonylation step to yield the target compound. This is particularly valuable in PET-tracer synthesis where the unlabelled substrates should be combined with the labeled precursor as late as possible in the reaction sequence, in order to decrease synthesis-time and thus optimize the uncorrected radiochemical yield.

When compounds are labeled with ¹¹C, it is usually important to maximize specific radioactivity. In order to achieve this, the isotopic dilution and the synthesis time must be minimized. Isotopic dilution from atmospheric carbon dioxide may be substantial when [¹¹C]carbon dioxide is used in a labeling reaction. Due to the low reactivity and atmospheric concentration of carbon monoxide (0.1 ppm vs. 3.4 × 10⁴ ppm for CO₂), this problem is reduced with reactions using [¹¹C]carbon monoxide.

The synthesis of [¹¹C]carbon monoxide from [¹¹C]carbon dioxide using a heated column containing reducing agents such as zinc, charcoal or molybdenum has been described previously in several publications. Although [¹¹C]carbon monoxide was one of the first ¹¹C-labelled compounds to be applied in tracer experiments in human, it has until recently not found any practical use in the production of PET-tracers. One reason for this is the low solubility and relative slow reaction rate of [¹¹C]carbon monoxide which causes low trapping efficiency in reaction media. The general procedure using precursors such as [¹¹C]methyl iodide, [¹¹C]hydrogen cyanide or [¹¹C]carbon dioxide is to transfer the radioactivity in a gas-phase, and trap the radioactivity by leading the gas stream through a reaction medium. Until recently this has been the only accessible procedure to handle [¹¹C]carbon monoxide in labeling synthesis. With this approach, the main part of the labeling syntheses with [¹¹C]carbon monoxide can be expected to give a very low yield or fail completely.

There are only a few examples of practically valuable ¹¹C-labelling syntheses using high pressure techniques (> 300 bar). In principal, high pressures can be utilized for increasing reaction rates and minimizing the amounts of reagents. One problem with this approach is how to confine the labeled precursor in a small high-pressure reactor. Another problem is the construction of the reactor. If a common column type of reactor is used (i.e. a cylinder with tubing attached to each end), the gas-phase will actually become efficiently excluded from the liquid phase at pressurization. The reason is that the gas-phase, in contracted from, will escape into the attached tubing and away from the bulk amount of the liquid reagent.

The cold-trap technique is widely used in the handling of ¹¹C-labelled precursors, particularly in the case of [¹¹C]carbon dioxide. The procedure has, however, only been performed in one single step and the labeled compound was always released in a continuous gas-stream simultaneous with the heating of the cold-trap. Furthermore, the volume of the material used to trap the labeled compound has been relative large in relation to the system to which the labeled compound has been transferred. Thus, the option of using this technique for radical concentration of the labeled compound and miniaturization of synthesis systems has not been explored. This is especially noteworthy in view of the fact that the amount of a ¹¹C-labelled compound usually is in the range 20-60 nmol.

Recent technical development for the production and use of [¹¹C] carbon monoxide has made this compound useful in labeling synthesis. WO 02/102711. describes a system and a method for the production and use of a carbon-isotope monoxide enriched gas-mixture from an initial carbon-isotope dioxide gas mixture. [¹¹C] carbon monoxide may be obtained in high radiochemical yield from cyclotron produced [¹¹C] carbon dioxide and can be used to yield target compounds with high specific radioactivity. This reactor overcomes the difficulties listed above and is useful in synthesis of ¹¹C-labelled compounds using [¹¹C] carbon monoxide in palladium or selenium mediated reaction as described by Kihlberg et al, J.Label.Comp.Radiopharm. 2001, S990-S992. With such method, a broad array of carbonyl compounds can be labelad (Kilhlberg, T.; Langstrom, B. J., Org. Chem. 1999, 9201-9205). The use of transition metal mediated reactions is, however, restricted by problems related to the competing β-hydride elimination reaction, which excludes or at least severely restricts utilization of organic electrophiles having hydrogen in β-position. Thus, a limitation of the transition metal mediated reactions is that most alkyl halides could not be used as substrates due to the β-hydride elimination reaction. One way to circumvent this problem is to use free-radical chemistry based on light irradiation of alkyl halides. We earlier succeeded in using free-radical chemistry for the carbonylation of alkyl iodides using amines to yield labeled amides WO 2005/042441

However, the attempt to yield labeled esters and acids in an analogous way (using alcohols and water as reactants instead of amines) is challenged by the low reactivity of alcohols and water in these reaction conditions (typically the yields of esters and acids compared to those of amides are lower by 10 to 100 times). One approach to solve this problem is to employ strong bases to promote these reactions. Good yields were achieved using THF as solvent, but only in the presence of a base (Itsenko et al., J. Org. Chem. 2005, 2244-2249). Similarly, in a photoinitiated method to obtain ¹²C esters from carbon monoxide, an alkyl or aryl iodide and alcohol, a base was required for the method to work (Nagatara et al., J. Am. Chem. Soc. 1997, 5465-5466. However bases may induce side reactions, such as elimination from alkyl iodides or substitution, decreasing the yield of radiolabeling reaction. Another problem is the difficulties with handling reaction mixtures because they may lose homogeneity. Therefore, there is a need for a milder method in order to use photo-induced free radical carbonylation with weakly reacting alcohols and water and provide target structures with high yield to further increase the utility of [¹¹C] carbon monoxide in preparing useful PET tracers.

Discussion or citation of a reference herein shall not be construed as an admission that such reference is prior art to the present invention.

### Summary of the Invention

The present invention provides a method for laboling syntheseis, consisting of
(a) providing a UV reactor assembly comprising a high pressure reaction chamber, a UV spot light source with a light guide, wherein the light guide is used to provide photo irradiation of a reaction mixture through a window in the reaction chamber,
(b) dissolving an alcohol and a photosensitizer,
(c) adding an alkyl or aryl iodide to the solution of step (b) to give a reagent volume to be labeled,
(d) introducing a carbon-isotope monoxide enriched gas-mixture into the reaction chamber of the UV reactor assembly via the gas inlet,
(e) introducing at high-pressure said reagent volume into the reaction chamber via the liquid inlet,
(f) turning on the UV spot tight source and waiting a predehmnincd time while the labeling synthesis occur, and
(g) collecting labeled ester from the reaction chamber,

The present invention provides a method for the synthesis of labeled esters using photo-initiated carbonylation with [¹¹C] carbon monoxide using alcohols photosensitizers and alkyl or aryl iodides.

### Brief Description of the Figures

Fig.1 shows a flow chart over the method according to the invention
Fig. 2 is a schematic view of a carbon-isotope monoxide production and labeling-system according to the invention.
Fig. 3 is the cross-sectional view of the reaction chamber.
Fig. 4 is a view of the UV spot light source.
Fig. 5 shows how the reaction chamber, magnetic stirrer, and the UV spot light source are arranged into the UV reactor assembly.
Fig. 6a and 6b show alternative embodiments of a reaction chamber according to the invention.

### Detailed Description of the Invention

The main advantage of the present invention is to overcome the limitations of transition metal-mediated reaction to synthesize ¹¹C-labeled esters using alkyl/aryl iodides and alcohols as precursors. The levels of specific radioactivity are high compared with alternative methods such as the use of Grignard reactions for preparation of [carbonyl-¹¹C] esters. Iodides used in this invention have a formula RI, where R is linear or cyclic alkyl or substituted alkyl, aryl or substituted aryl, and may contain fluoro, ester and carboxyl groups, which are separated by at least one carbon atom from the carbon atom bearing the iodide atom. Alcohol used may be a primary or secondary general formula being R'OH, where R' is linear or cyclic alkyl or substituted alkyl, and may contain fluoro, ester and carboxyl groups.

Photosensitization is the process by which a photochemical or photophysical alteration occurs in one molecular entity as a result of initial absorption of radiation by another molecular entity called a photosensitizer (IUPAC Compendium of Chemical Terminology, 1997). In a preferred embodiment, photosensitizers are aliphatic or aromatic ketones or aryl amines. Examples of photosensitizers include acetone, benzaphenone,and xanthone, and triphenylamine. Further examples of photosensitizers are provided in Handbook of Photochemistry, by S. L. Murov, I. Carmichael and G. Hug, 2nd cd., Marcel Dekker, Inc., New York (1993).

The resultant labeled esters have a formula 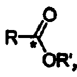 wherein R and R' are defined as above. They and their pharmaceutically acceptable salts and/or solvates thereof provide valuable PET tracers in various PET studies.

The term "phamaceutically acceptable" means compatible with the treatment of animals, in particular, humans.

The term "pharmaceutically acceptable salt" refers to salt forms that are pharmacologically suitable for or compatible with the treatment of patients.

The term "solvate" as used herein means a compound wherein molecules of a suitable solvent are incorporated in the crystal lattice. A suitable solvent is physiologically tolerable at the dosage administered. Examples of suitable solvents are ethanol and water. When water is the solvent, the molecule is referred to as a "hydrate".

General reaction scheme for the synthesis of labeled esters are as illustrated below: 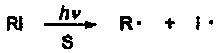 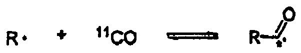 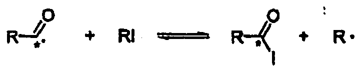 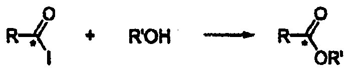 wherein R and R' are as defined above, S is a photosensitizer. * indicates the ¹¹C-labeled position.

The radiolabelled compounds, or pharmaceutically acceptably salts and solvates thereof, are suitably formulated into pharmaceutical or radiopharmaceutical compositions for administration to human subjects in a biologically compatible form suitable for administration *in vivo.*

The term an "effective amount" as used herein is that amount sufficient to effect desired results, including clinical results, and, as such, an "effective amount" depends upon the context in which it is being applied

The term "subject" as used herein includes all members of the animal kingdom including human. The subject is preferably a human.

The labelled compounds of the present invention are useful in a method for conducting positron emission tomography of a subject comprising administering to the subject an effective amount of a radiolabelled compound, or pharmaceutically acceptable salts and solvates thereof; of the instant invention and measuring the distribution within the subject of the compound by PET.

The radiolabeled compounds of the invention may be administered to a patient in a variety of forms depending on the selected route of administration, as will be understood by those skilled in the art. The compositions are preferably administered by intraveneous administration, and the radiopharmaceutical compositions formulated accordingly, for example together with any physiologically and radiologically tolerable vehicle appropriate for administering the compound systemically.

In a preferred embodiment of the instant invention, it provides a method which is nearly quantitative conversion of carbon-isotope monoxide into labeled products can be accomplished.

There are several other advantages with the present method. The high-pressure technique makes it possible to use low boiling solvents such as diethyl ether at high temperatures (e.g 200 °C). The use of a closed system consisting of materials that prevents gas diffusion, increases the stability of sensitive compounds and could be advantageous also with respect to Good Manufacturing Practice (GMP).

Still other advantages are achieved in that the resulting labeled compound is highly concentrated, and that the miniaturization of the synthesis system facilitates automation, rapid synthesis and purification, and optimization of specific radioactivity through minimization of isotopic dilution.

Most important is the opening of completely new synthesis possibilities, as exemplified by the present invention.

Embodiments of the invention will now be described with reference to the figures.

The term carbon-isotope that is used throughout this application refers to ¹¹C, but it should be understood that ¹¹C may be substituted by other carbon-isotopes, such as ¹³C and ¹⁴C, if desired. However, the present claimed method is restricted to ¹¹C.

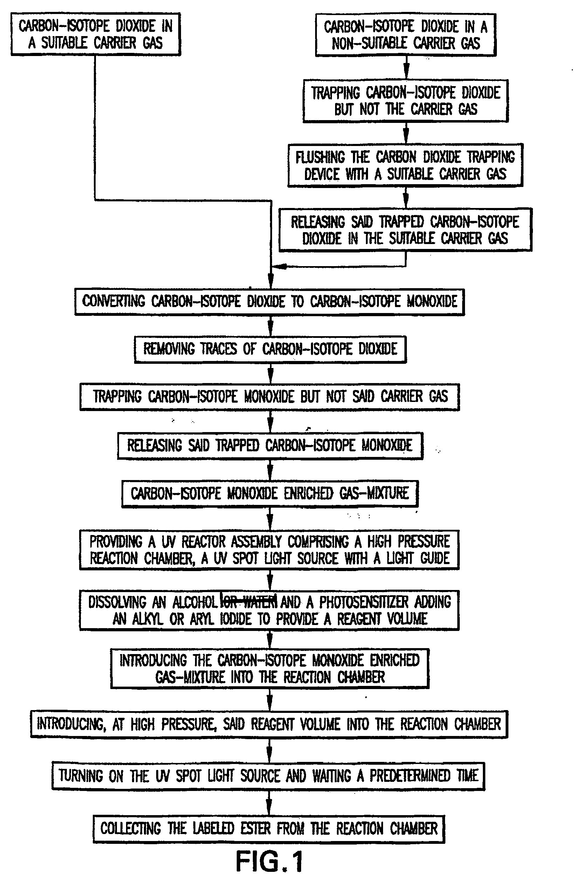

Fig. 1 shows a flow chart over the method according to the invention, which firstly comprises production of a carbon-isotope monoxide enriched gas-mixtnre and secondly a labeling synthesis procedure. More in detail the production part of the method comprises the steps of:
- Providing carbon-isotope dioxide in a suitable carrier gas of a type that will be described in detail below.
- Converting carbon-isotope dioxide to carbon-isotope monoxide by introducing said gas mixture in a reactor device which will be described in detail below.
- Removing traces of carbon-isotope dioxide by flooding the converted gas-mixture through a carbon dioxide removal device wherein carbon-isotope dioxide is trapped but not carbon-isotope monoxide nor the carrier gas. The carbon dioxide removal device will be described in detail below.
- Trapping carbon-isotope monoxide in a carbon monoxide trapping device, wherein carbon-isotope monoxide is trapped but not said carrier gas. The carbon monoxide trapping device will be described in detail below.
- Releasing said trapped carbon-isotope monoxide from said trapping device, whereby a volume of carbon-isotope monoxide enriched gas-mixture is achieved

The production step may further comprise a step of charging carrier gas for the initial carbon isotope dioxide gas mixture if the initial carbon-isotope dioxide gas mixture is comprised of carbon-isotope dioxide and a first carrier gas not suitable as carrier gas for carbon monoxide due to similar molecular properties, such as nitrogen. More in detail the step of providing carbon-isotope dioxide in a suitable second carrier gas such as He, Ar, comprises the steps of:
- Flooding the initial carbon-isotope dioxide gas mixture through a carbon dioxide trapping device, wherein carbon-isotope dioxide is trapped but not said first carrier gas. The carbon dioxide trapping device will be described in detail below.
- Flushing said carbon dioxide trapping device with said suitable second carrier gas to remove the remainders of said first carrier gas.
- Releasing said trapped carbon-isotope dioxide in said suitable second carrier gas.

The labeling synthesis step that follows the production step utilizes the produced carbon-isotope dioxide enriched gas-mixture as labeling reactant. More in detail the step of labeling synthesis comprises the steps of
- Providing a UV reactor assembly comprising a UV spot light source and a high pressure reaction chamber having a liquid reagent inlet and a labeling reactant inlet in a bottom surface thereof In a preferred embodiment, the LAV reactor assembly further comprises a magnetic stirrer and a magnetic stirring bar. In another preferred embodiment, the UV reactor assembly further comprises a protective housing and a bench where the reaction chamber, UV spot light guide and the magnetic stirrer can be mounted. The UV reactor assembly and the reaction chamber will be described in detail below.
- Providing a reagent volume that is to be labeled. The reagent volume can be prepared in following steps: 1. Dissolve an alcohol and a photosensitizer; 2. Add alkyl or aryl iodide to the solution of step 1 to form a reagent volume as late as possible before being introduced into the high pressure reaction chamber. Definition and examples of photosensitizers are provided above.
- Introducing the carbon-isotope monoxide enriched gas-mixture into the reaction chamber via the labeling reactant inlet.
- Introducing, at high pressure, said liquid reagent into the reaction chamber via the liquid reagent inlet.
- Turning on the UV spot light source and waiting a predetermined time while the labeling synthesis occurs.
- Collecting the solution of labeled ester from the reaction chamber.

The step of waiting a predetermined time may further comprise adjusting the temperature of the reaction chamber such that the labeling synthesis is enhanced.

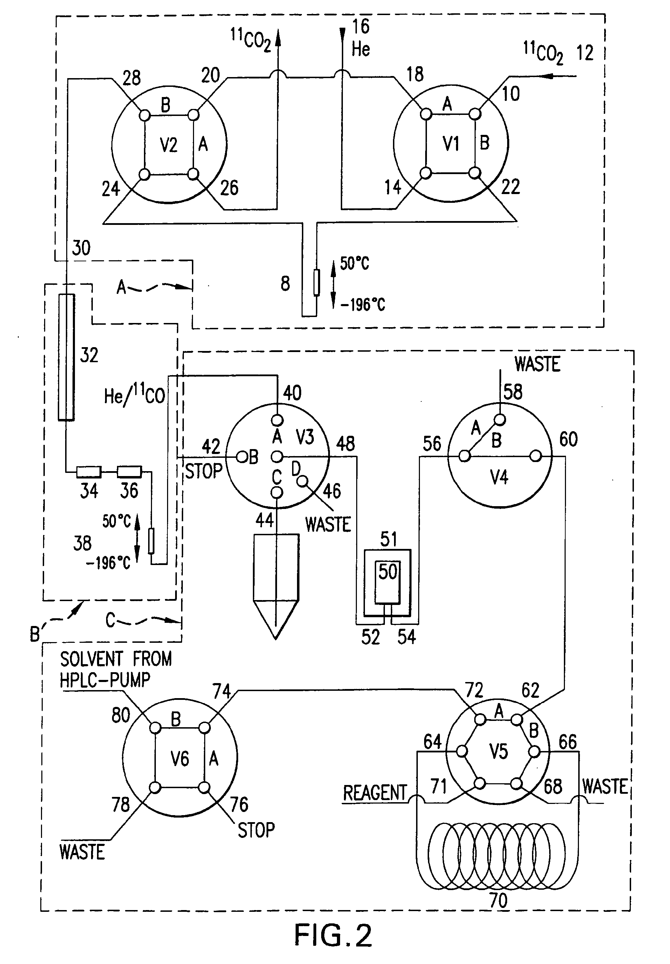

Fig. 2 schematically shows a [¹¹C]carbon dioxide production and labeling-system used in the present invention. The system is comprised of three main blocks, each handling one of the three main steps of the method of production and labeling:
Block A is used to perform a change of carrier gas for an initial carbon-isotope dioxide gas mixture, if the initial carbon-isotope dioxide gas mixture is comprised of carbon-isotope dioxide and a first carrier gas not suitable as carrier gas for carbon monoxide.
Block B is used to perform the conversion from carbon-isotope dioxide to carbon-isotope monoxide, and purify and concentrate the converted carbon-isotope monoxide gas mixture.
Block C is used to perform the carbon-isotope monoxide labeling synthesis.

Block A is normally needed due to the fact that carbon-isotope dioxide usually is produced using the 14N(p,α)¹¹C reaction in a target gas containing nitrogen and 0.1% oxygen, bombarded with 17 MeV protons, whereby the initial carbon-isotope dioxide gas mixture comprises nitrogen as carrier gas. However, compared with carbon monoxide, nitrogen show certain similarities in molecular properties that makes it difficult to separate them from mach other, e.g. in a trapping device , whereby it is difficult to increase the concentration of carbon-isotope monoxide in such a gas mixture. Suitable carrier gases may instead be helium or argon. Block A can also used to change the pressure of the carrier gas (e.g. from 1 to 4 bar), in case the external system does not tolerate the gas pressure needed in block B and C. In an alternative embodiment the initial carbon-isotope dioxide gas mixture is comprised of carbon-isotope dioxide and a first carrier gas that is well suited as carrier gas for carbon monoxide, whereby the block A may be simplified or even excluded.

According to a preferred embodiment used in the present application (Fig. 2), block A is comprised of a first valve V1, a carbon dioxide trapping device 9, and a second valve V2.

The first valve V1 has a carbon dioxide inlet 10 connected to a source of initial carbon-isotope dioxide gas mixture 12, a carrier gas inlet 14 connected to a source of suitable carrier gas 16, such as helium and argon. The first valve V1 further has a first outlet 18 connected to a first inlet 20 of the second valve V2, and a second outlet 22 connected to the carbon dioxide trapping device 8. The valve V1 may bc operated in two modes A, B, in mode A the carbon dioxide inlet 10 is connected to the first outlet 18 and the carrier gas inlet 14 is connected to the second outlet 22, and in mode B the carbon dioxide inlet 10 is connected to the second outlet 22 and the carrier gas inlet 14 is connected to the first outlet 18.

In addition to the first inlet 20, the second valve V2 has a second inlet 24 connected to the carbon dioxide trapping device 8. The second valve V2 further has a waste outlet 26, and a product outlet 28 connected to a product inlet 30 of block B. The valve V2 may be operated in two modes A. B, in mode A the first inlet 20 is connected to the waste outlet 26 and the second inlet 24 is connected to the product outlet 28, and in mode B the first inlet 20 is connected to the product outlet 28 and the second inlet 24 is connected to the waste outlet 26.

The carbon dioxide trapping device 8 is a device wherein carbon dioxide is trapped but not said first carrier gas, which trapped carbon dioxide thereafter may be released in a controlled manner, This may preferably be achieved by using a cold trap, such as a column containing a material which in a cold state, (e.g. -196°C as in liquid nitrogen or -186 °C as in liquid argon) selectively trap carbon dioxide and in a warm state (e.g. +50°C) releases the trapped carbon dioxide. (In this text the expression "cold trap" is not restricted to the use of cryogenics. Thus, materials that trap the topical compound at room temperature and release it at a higher temperature are included). One suitable material is porapac Q®. The trapping behavior of a porapao-column is related to dipole-dipole interactions or possibly Van der Waal interaktions. The said column 8 is preferably formed such that the volume of the trapping material is to be large enough to efficiently trap (>95%) the carbon-isotope dioxide, and small enough not to prolong the transfer of trapped carbon dioxide to block B. In the case of porapac Q® and a flow of 100 ml nitrogen/ min, the volume should be 50-150 µl. The cooling and heating of the carbon dioxide trapping device 8 may further be arranged such that it is performed as an automated process, e.g. by automatically lowering the cohumn into liquid nitrogen and moving it from there into a heating arrangement.

According to the preferred embodiment of Fig. 2 block B used in the present application is comprised of a reactor device 32 in which catbon-isotope dioxide is converted to carbon-isotope monoxide, a carbon dioxide removal device 34, a check-valve 36, and a carbon monoxide trapping device 38, which all are connected in a line.

In the preferred embodiment the reactor device 32 is a reactor furnace comprising a material that when heated to the right temperature interval converts carbon-isotope dioxide to carbon-isotope monoxide. A broad range of different materials with the ability to convert carbon dioxide into carbon monoxide may be used, e.g. zinc or molybdenum or any other element or compound with similar reductive properties. If the reactor device 32 is a zinc furnace it should be heated to 400°C, and it is important that the temperature is regulated with high precision. The melting point of zinc is 420 °C and the zinc-furnace quickly loses it ability to transform carbon dioxide into carbon monoxide when the temperature reaches over 410 °C, probably due to changed surface properties. The material should be efficient in relation to its amount to ensure that a small amount can be used, which will minimize the time needed to transfer radioactivity from the carbon dioxide trapping device 8 to the subsequent carbon monoxide trapping device 38. The amount of material in the furnace should be large enough to ensure a practical life-time for the furnace (at least several days). In the case of zinc granulates, the volume should be 100-1000 µl.

The carbon dioxide removal device 34 is used to remove traces of carbon-isotope dioxide from the gas mixture exiting the reactor device 32. In the carbon dioxide removal device 34, carbon-isotope dioxide is trapped but not carbon-isotope monoxide nor the carrier gas. The carbon dioxide removal device 34 may be comprised of a column containing ascarite® (i.e. sodium hydroxide on silica). Carbon-isotope dioxide that has not reacted in the reactor device 32 is trapped in this column (it reacts with sodium hydroxide and turns into sodium carbonate), while carbon-isotope monoxide passes through. The radioactivity in the carbon dioxide removal device 34 is monitored as a high value indicates that the reactor device 32 is not functioning properly.

Like the carbon dioxide trapping device 8, the carbon monoxide trapping device 38, has a trapping and a releasing state. In the trapping state carbon-isotope monoxide is selectively trapped but not said carrier gas, and in the releasing state said trapped carbon-isotope monoxide is released in a controlled manner. This may preferably be achieved by using a cold trap, such as a column containing silica which selectively trap carbon monoxide in a cold state below -100°C, e.g. -196°C as in liquid nitrogen or -186 °C as in liquid argon, and releases the trapped carbon monoxide in a warm state (e.g. +50°C). Like the porapac-column, the trapping behavior of the silica-column is related to dipole-dipole interactions or possibly Van der Waal interactions. The ability of the silica-column to trap carbon-isotope monoxide is reduced if the helium, carrying the radioactivity, contains nitrogen. A rationale is that since the physical properties of nitrogen are similar to carbon monoxide, nitrogen competes with carbon monoxide for the trapping sites on the silica.

According to the preferred embodiment of Fig. 2, block C is comprised of a first and a second reaction chamber valve V3 and V4, a reagent valve V5, an injection loop 70 and a solvent valve V6, and the UV reactor assembly 51 which comprises a UV lamp 91, a concave minor 92 and a reaction chamber 50.

The first reaction chamber valve V3 has a gas mixture inlet 40 connected to the carbon monoxide trapping device 38, a stop position 42, a collection outlet 44, a waste outlet 46, and a reaction chamber connection port 48 connected to a gas inlet 52 of the reaction chamber 50. The first reaction chamber valve V3 has four modes of operation A to D. The reaction chamber connection port 48 is: in mode A connected to the gas mixture inlet 40, in mode B connected to the stop position 42, in mode C connected to the collection outlet 44, and in mode D connected to the waste outlet 46.

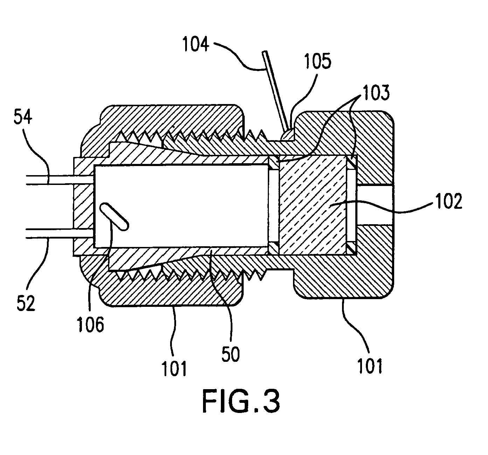

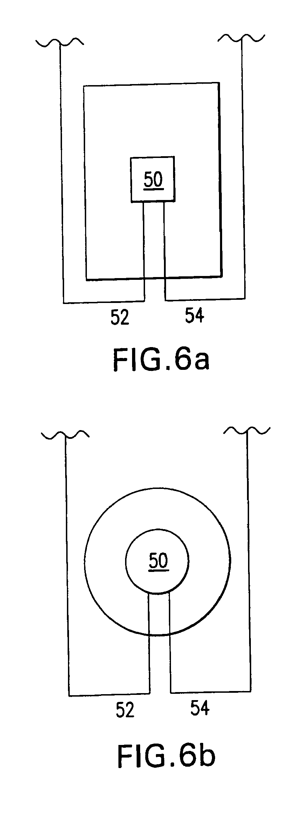

Fig. 3 shows the reaction chamber 50 (micro-autoclave) which has a gas inlet 52 and a liquid inlet 54, which are arranged such that they terminate at the bottom surface of the chamber. Gas inlet 52 may also be used as product outlet after the labeling is finished. During operation the carbon-isotope monoxide enriched gas mixture is introduced into the reaction chamber 50 through the gas inlet 52, where after the liquid reagent at high pressure enters the reaction chamber 50 through the liquid inlet 54. Fig. 6a and 6b shows schematic views of two preferred reaction chambers 50 in cross section. Fig 6a is a cylindrical chamber which is fairly easy to produce, whereas the spherical chamber of fig. 6b is the most preferred embodiment, as the surface area to volume-ratio of the chamber is further minimized. A minimal surface area to volume-ratio optimizes the recovery of labeled product and minimizes possible reactions with the surface material. Due to the "diving-bell construction" of the reaction chamber 50, both the gas inlet 52 and the liquid inlet 54 becomes liquid-filled and the reaction chamber 50 is filled from the bottom upwards. The gas-volume containing the carbon-isotope monoxide is thus trapped and given efficient contact with the reaction mixture. Since the final pressure of the liquid is 80 times higher than the original gas pressure, the final gas volume will be less than 2 % of the liquid volume according to the general gas-law. Thus, a pseudo one phase system will result. In the instant application, the term "pseudo one phase system" means a closed volume with a small surface area to volume-ratio combining >96% liquid and <4% gas at pressures exceeding 200 ban. In most syntheses the transfer of carbon monoxide from the gas-phase to the liquid phase will probably not be the rate limiting step. After the labeling is finished the labeled volume is nearly quantitatively transferred from the reaction chamber by the internal pressure via the gas inlet/product outlet 52 and the first reaction chamber valve V3 in position C.

In a specific embodiment. Fig. 3 shows a reaction chamber made from stainless steel (Vako^{™})^{®} colmn end fitting 101. It is equipped with sapphire window 102, which is a hard material transparent to short wavelength UV radiation. The window is pressed between two Teflon washers 103 inside the drilled column end fitting to make the reactor tight at high pressures. Temperature measurement can be accomplished with the thermocouple 104 attached by solder drop 105 to the outer side of the reactor. A magnet stirrer (not shown) drives small Teflon coated magnet stirring bar 106 placed inside the reaction chamber. The magnetic stirrer can be attached against the bottom of the reaction chamber. Distance between the magnet : stirrer and the reactor should be minimal.

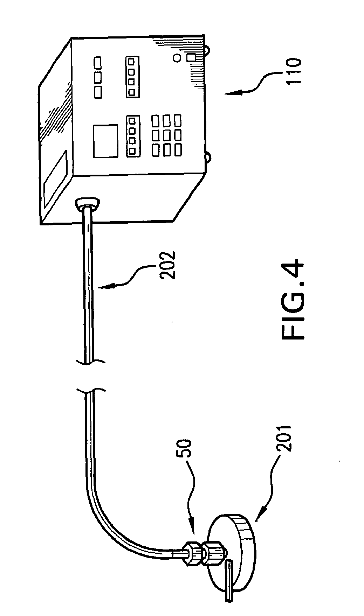

Fig. 4 shows a commercial UV spot light source 110 (for example, Hamamatsu Lightningcure^{™} LC5)^{®}, which is an example of UV spot light sources that can be used in the instant invention. Light source 110 has necessary means of operating and controlling the photo irradiation that is produced, of the light source is available from the manufacturer (Hamamatsu Photonics K.K.). Thus intensity and time duration of the photo irradiator are easily adjusted by an operator. Light source 110 may be externally controlled by a computer, providing a possibility for automating the reactor assembly. The photo irradiation is delivered to the reaction vessel through a flexible light guide, which is an accessory of Hamamatsu laghtningcure LC5^{®}. Thus light source 110 may be placed at the distance from the reaction chamber providing the possibility to save precious space inside a sheltered hot-cell, where the radiolabeling syntheses are carried out. Light source 110 complies with the existing industrial safety standards. Further, optional accessories (e.g. changeable lamps, optical filters) are provided which may be advantageously used for adjusting the properties of the photo irradiation.

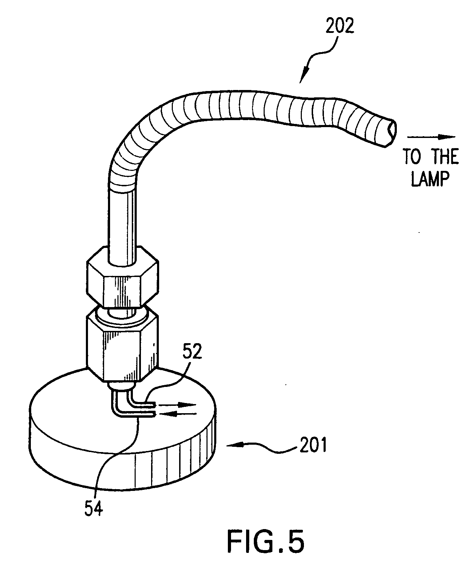

Fig. 5 shows the reaction chamber 50 situated a magnetic stirrer 201, with gas inlet/product outlet 52 and liquid inlet 54 facing the magnetic stirrer 201. Top of the reaction chamber 50 is connected through the flexible light guide 202 to the UV spot light source (not shown).

Referring back to Fig. 2, the second reaction chamber valve V4 has a reaction chamber connection port 56, a waste outlet 58, and a reagent inlet 60. The second reaction chamber valve V4 has two modes of operation A and B. The reaction chamber connection port 56 is: in mode A connected to the waste outlet 58, and in mode B it is connected to the reagent inlet 60.

The reagent valve V5, has a reagent outlet 62 connected to the reagent inlet 60 of the second reaction chamber valve V4, an injection loop inlet 64 and outlet 66 between which the injection loop 70 is connected, a waste outlet 68, a reagent inlet 71 connected to a reagent source, and a solvent inlet 72. The reagent valve V5, has two modes of operation A and B. In mode A the reagent inlet 71 is connected to the injection loop inlet 64, and the injection loop outlet 66 is connected to the waste outlet 68, whereby a reagent may be fed into the injection loop 70. In mode B the solvent inlet 72 is connected to the injection loop inlet 64, and the injection loop outlet 66 is connected to the reagent outlet 62, whereby reagent stored in the injection loop 70 may be forced via the second reaction chamber valve V4 into the reaction chamber 50 if a high pressure is applied on the solvent inlet 72.

The solvent valve V6, has a solvent outlet 74 connected to the solvent inlet 72 of the reagent valve V5, a stop position 76, a waste outlet 78, and a solvent inlet 80 connected to a solvent supplying HPLC-pump (High Performance Liquid Chromatography) or any liquid-pump capable of pumping organic solvents at 0-10 ml/ min at pressures up to 400 bar (not shown). The solvent valve V6, has two modes of operation A and B. In mode A the solvent outlet 74 is connected to the stop position 76, and the solvent inlet 80 is connected to the waste outlet 78. In mode B the solvent outlet 74 is connected to the solvent inlet 80, whereby solvent may be pumped into the system at high pressure by the HPLC-pump.

Except for the small volume of silica in the carbon monoxide trapping devise 38, an important difference in comparison to the carbon dioxide trapping device 8, as well as to all related prior art, is the procedure used for releasing the carbon monoxide. After the trapping of carbon monoxide on carbon monoxide trapping devise 8, valve V3 is changed from position A to B to stop the flow from the carbon monoxide trapping devise 38 and increase the gas-pressure on the carbon monoxide trapping devise 38 to the set feeding gas pressure (3-5 bar). The carbon monoxide trapping devise 38 is then heated to release the carbon monoxide from the silica surface while not significantly expanding the volume of carbon monoxide in the carrier gas. Valve V4 is changed from position A to B and valve V3 is then changed from position B to A. At this instance the carbon monoxide is rapidly and almost quantitatively transferred in a well-defined micro-plug into the reaction chamber 50. Micro-plug is defined as a gas volume less than 10% of the volume of the reaction chamber 50, containing the topical substance (e.g. 1-20 µL). This unique method for efficient mass-transfer to a small reaction chamber 50, having a closed outlet, has the following prerequisites:
- A micro-column 38 defined as follows should be used. The volume of the trapping material (e.g. silica) should be large enough to efficiently trap (>95%) the carbon-isotope monoxide, and small enough (< 1% of the volume of a subsequent reaction chamber 50) to allow maximal concentration of the carbon-isotope monoxide. In the case of silica and a reaction chamber 50 volume of 200 µl, the silica volume should be 0.1-2 µl.
- The dead volumes of the tubing and valve(s) connecting the silica column and the reaction chamber 50 should be minimal (<10% of the micro-autoclave volume).
- The pressure of the carrier gas should be 3-5 times higher than the pressure in the reaction chamber 50 before transfer (101.32501kPa; 1 atm.).

In one specific preferred embodiment specifications, materials and components are chosen as follows. High valve from Valco®, Reodyne® or Cheminert® are used. Stainless steel tubing with o.d. 1/16" is used except for the connections to the porapac-column 8, the silica-column 38 and the reaction chamber 50 where stainless steel tubing with o.d. 1/32" are used in order to facilitate the translation movement. The connections between V1, V2 and V3 should have an inner diameter of 0.2-1 mm. The requirement is that the inner diameter should be large enough not to obstruct the possibility to achieve the optimal flow of He (2-50 ml/ min) through the system, and small enough not to prolong the time needed to transfer the radioactivity from the porapac-column 8 to the silica-column 38. The dead volume of the connection between V3 and the autoclave should be minimized (< 10% of the autoclave volume). The inner diameter (0.05-0.1 mm) of the connection must be large enough to allow optimal He flow (2-50 ml/ min). The dead volume of the connection between V4 and V5 should be less than 10% of the autoclave volume.

The porapac-column 8 preferably is comprised of a stainless steel tube (o.d.= 1/8", i.d.= 2 mm, 1= 20 mm) filled with Porapac Q® and fitted with stainless steel screens. The silica-column 38 preferably is comprised of a stainless steel tube (o.d= 1/16", i.d.= 0.1 mm) with a cavity (d=1 mm, h= 1 mm, V= 0.8 µl) in the end. The cavity is filled with silica powder (100/80 mesh) of GC-stationary phase type. The end of the column is fitted against a stainless steel screen.

It should be noted that a broad range of different materials could be used in the trapping devices. If a GC-material is chosen, the criterions should be good retardation and good peak-shape for carbon dioxide and carbon monoxide respectively. The latter will ensure optimal recovery of the radioactivity.

Below a detailed description is given of a method of producing carbon-isotope using an exemplary system as described above.

Preparations of the system are performed by the steps 1 to 5:
1. V1 in position A, V2 in position A, V3 in position A, V4 in position A, helium flow on with a max pressure of 5 bar. With this setting, the helium flow goes through the porapac column, the zinc furnace, the silica column, the reaction chamber 50 and out through V4. The system is conditioned, the reaction chamber 50 is rid of solvent and it can be checked that helium can be flowed through the system with at least 10 ml/min. UV lamp 91 is turned on.
2. The zine-furnace is turned on and set at 400 °C.
3. The porapac and silica-columns are cooled with liquid nitrogen, At-196 °C, the porapac and silica-column efficiently traps carbon-isotope dioxide and carbon-isotope monoxide respectively.
4. V5 in position A (load). The injection loop (250 µl), attached to V5, is loaded with the reaction mixture.
5. The HPLC pump is attached to a flask with freshly distilled THF (or other high quality solvent) and primed. V6 in position A.

Production of carbon-isotope dioxide may be performed by the steps 6 to 7:
6. Carbon-isotope dioxide is produced using the 14N(p,α)¹¹C reaction in a target gas containing nitrogen (AGA, Nitrogen 6.0) and 0.1% oxygen (AGA. Oxygen 4.8), bombarded with 17 MeV protons.
7. The carbon-isotope dioxide is transferred to the apparatus using nitrogen with a flow of 100 ml/min.

Synthesis of carbon-isotope may thereafter be performed by the steps 8 to 16
8. V1 in position B and V2 in position B. The nitrogen flow containing the carbon-isotope dioxide is now directed through the porapac-cohmm (cooled to -196 °C) and out through a waste line. The radioactivity trapped In the porapac-column is monitored.
9. When the radioactivity has peaked, V1 is changed to position A. Now a helium flow is directed through the porapac-column and out through the waste line. By this operation the tubings and the porapac-column arc rid of nitrogen.
10. V2 in position A and the porapac-columm is warmed to 50 °C. The radioactivity is now released from the porapac-column and transferred with a helium flow of 10 ml/ min into the zinc-furnace where it is transformed into carbon-isotope monoxide.
11. Before reaching the silica-column (cooled to -196 °C), the gas flow passes the ascarite-column. The carbon-isotope monoxide is now trapped on the silica-column. The radioactivity in the silica-column is monitored and when the value has peaked, V3 is set to position B and then V4 is set to position B.
12. The silica-column is heated to 50 °C, which releases the carbon-isotope monoxide. V3 is set to position A and the carbon-isotope monoxide is transferred to the reaction chamber 50 within 15 s.
13. V3 is set to position B, V5 is set to position B, the HPLC-pump is turned on (flow 7 ml/ min) and V6 is set to position B. Using the pressurised THF (or other solvent), the reaction mixture is transferred to the reaction chamber 50. When the HPLC-pump has reached its set pressure limit (e.g 40 Mpa), it is automatically turned off and the V6 is set to position A.
14. UV spot light source 110, magnetic stirrer 201 and magnet stirring bar 106 in reaction chamber 50 are turned on.
15. After a sufficient reaction-time (usually 5 min), V3 is set to position C and the content of the reaction chamber 50 is transferred to a collection vial.
16. The reaction chamber 50 can be rinsed by the following procedure: V3 is set to position B, the HPLC-pump is turned on, V6 is set to position B and when maximal pressure is reached V6 is set to position A and V3 is set to position 3 thereby transferring the rinse volume to the collection vial.

With the recently developed fully automated version of the reaction chamber 50 system according to the invention, the value of [¹¹C]carbon monoxide as a precursor for ¹¹C-labelled tracers has become comparable with [¹¹C]methyl iodide. Currently, [¹¹C]methyl iodide is the most frequently used ¹¹C-precursor due to ease in production and handling and since groups suitable for labeling with [¹¹C]methyl iodide (e.g. hetero atom bound methyl groups) are common among biologically active substances. Carbonyl groups, which can be conveniently labeled with [¹¹C]carbon monoxide, are also common among biologically active substances. In many cases, due to metabolic events in vivo, a carbonyl group may oven be more advantageous than a methyl group as labeling position. The use of [¹¹C]carbon monoxide for production of PET-tracers may thus become an interesting complement to [¹¹C]methyl iodide. Furthermore, through the use of similar technology, this method will be applicable for synthesis of ¹³C and ¹⁴C substituted compound.

### Examples

The invention is further described in the following examples .

### Example 1-Precursors and Resultant Products

Precursors that were used to label esters are shown in List. 1. 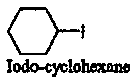 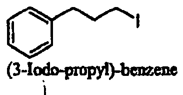 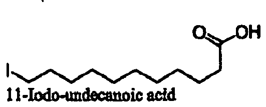 List 1a. Iodides used as precursors in the synthesis of labeled esters

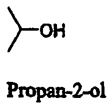

CH₃OH

Methanol
List 1b. Alcohols used as precursors in the synthesis of labeled esters

The following experiments illustrate the present invention. Radical carbonylation using submicromolar amounts of [¹¹C]carbon monoxide is performed yielding labeled esters shown in Table 1 as target compounds.

**Table 1. Radiochemical yields of labeled compounds.**

| Entry | Labelled compound^{a} | Solvents | Additive (mmol) | ¹¹CO conv. (%) | Yield^{b} (%) | Isolated Yield (%) | N^{c} |
|---|---|---|---|---|---|---|---|
| 1* | 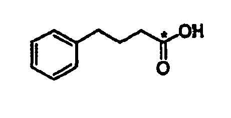 | Acetone/H₂O (4:1) | - | 91±3 | 73±3 | 62±3 | 3 |
| 2 | 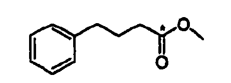 | Acetone/MeOH (1:1) | - | 86±1 | 85±1 | 56±1 | 3 |
| 3* | 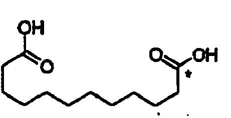 | Acetone/H₂O | - | 86±4 | 73±5 | 63±6 | 3 |
| 4 | 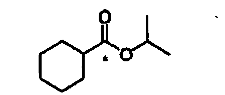 | THF/IPA^{d}(1:1) | triphenylamine, 0.1 | 47±3 | 79±1 | 38±3 | 2 |
| 5 | 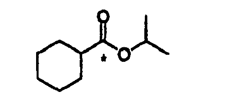 | THF/IPA (1:1) | benzophenone, 0.1 | 85 | 71 | 59 | 1 |
| 6 | 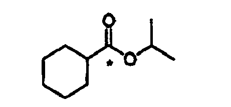 | Acetone/IPA | - | 85±2 | 80±3 | 67±1 | 2 |

| | | | | | | | |
|---|---|---|---|---|---|---|---|
| ^{a}The position of ¹¹C label is denoted by an asterisk. ^{b}Decay-correted radiochemical yield determined by LC. ^{c} Number of runs. ^{d} IPA is Propan-2-ol * technical information not belonging to the invention | | | | | | | |

### Example 2-Experimental Setup

[¹¹C]Carbon dioxide production was performed using a Scanditronix MC-17 cyclotron at Uppsala Imanet. The ¹⁴N(p,α)¹¹C reaction was employed in a gas target containing nitrogen (Nitrogen 6.0) and 0.1% oxygen (Oxygen 4.8), that was bombarded with 17 MeV protons.

[¹¹C]Carbon monoxide was obtained by reduction of [¹¹C]carbon dioxide as described previously (Kihlberg, T.; Langström, B. *Method and apparatus for production and use of [¹¹C] carbon monoxide in labeling synthesis.* Swedish Pending Patent Application No. 0102174-0).

Liquid chromatographic analysis (LC) was performed with a gradient pump and a variable wavelength UV-detector in series with a β⁺-flow detector. An automated synthesis apparatus, Synthia (Bjurling, P.; Reineck, R.; Westerberg, G.; Gee, A. D.; Sutcliffe, J., Langström, B. In Proceedings of the YIth workshop on targetry and target chemistry; TRIUMF: Vancouver, Canada, 1995; pp 282-284) was used for LC purification of the labelled products.

Radioactivity was measured in an ion chamber. Xenon-mercury lamp was used as a photo-irradiation source.

In the analysis of the ¹¹C-labeled compounds, isotopically unchanged reference substances were used for comparison in all the LC runs.

NMR spectra were recorded at 400 MHz for ¹H and at 100 MHz for ¹³C, at 25°C. Chemical shifts were referenced to TMS via the solvent signals.

LC-MS analysis was performed with electrospray ionization.

Alkyl iodides were commercially available or otherwise prepared from commercial alkyl bromides by the Finkelstein reaction.

### Example 3-Preparation of [carbonyl-¹¹C] esters

General procedure: An alcohol (200 µmol) and a photosensitizer (Table 1) were placed in a capped vial (1 mL, flushed beforehand with nitrogen to remove air) and dissolved in THF (500 µL); in some cases the alcohol was used as a solvent instead of THF. An iodide (100 µmol) was added to the solution ca 7 min before the start of the synthesis. The resulting mixture was pressurized (over 40 MPa) into the autoclave, pre-charged with [¹¹C]carbon monoxide (10⁻⁸ -10⁻⁹ mol) and helium gas mixture. The mixture was irradiated with the Xe-Hg lamp for 6 min with stirring at 35°C. The crude reaction mixture was then transferred from the autoclave to a capped vial, held under reduced pressure. After measurement of the radioactivity the vial was purged with nitrogen and the radioactivity was measured again. The crude product was diluted with acetonitrile or methanol (0.6 mL) and injected on the semi-preparative LC. Analytical LC and LC-MS were used to assess the identity and radiochemical purity of the collected fractions.

## Claims

1. A method for labeling synthesis, consisting of:
(a) providing a UV reactor assembly comprising a high pressure reaction chamber, a UV spot light source with a light guide, wherein the light guide is used to provide photo irradiation of a reaction mixture through a window in the reaction chamber,
(b) dissolving an alcohol and a photosensitizer,
(c) adding an alkyl or aryl iodide to the solution of step (b) to give a reagent volume to be labeled,
(d) introducing a [¹¹C]-carbon monoxide enriched gas-mixture into the reaction chamber of the UV reactor assembly via the gas inlet,
(e) introducing at high pressure said reagent volume into the reaction chamber via the liquid inlet,
(f) turning on the UV spot light source and waiting for a predetermined time while the labeling synthesis occur, and
(g) collecting the labeled ester from the reaction chamber.

2. A method of claim 1, wherein the [¹¹C]-carbon monoxide enriched gas-mixture is produced by a method comprising:
(a) providing [¹¹C]-carbon dioxide in a suitable carrier gas,
(b) converting [¹¹C]-carbon dioxide to [¹¹C]-carbon monoxide by introducing said gas mixture in a reactor device,
(c) trapping [¹¹C]-carbon monoxide in a carbon monoxide trapping device, wherein [¹¹C]-carbon monoxide is trapped but not said carrier gas, and
(d) releasing said trapped [¹¹C]-carbon monoxide from said trapping device in a well defined micro-plug, whereby a volume of [¹¹C]-carbon monoxide enriched gas-mixture is achieved.

3. A method of claim 1, wherein the step of introducing the reagent is performed using a pressure that is 80 times higher than the pressure before the introduction, in order to maintain a pseudo one-phase system wherein the "pseudo one phase system" means a closed volume with a small surface area to volume ratio containing more than 96% liquid and less than 4% gas at pressures exceeding 200 bar.

4. A method of claim 1, wherein the step of waiting a predetermined time comprises stirring in the reaction chamber to enhance the labeling synthesis.

5. A method of claim 4, wherein the step of waiting a predetermined time further comprises adjusting the temperature of the reaction chamber so that the labeling synthesis is enhanced.

6. A method of claim 1 wherein said alkyl or aryl iodide is a compound of formula (I) and said alcohol is an alcohol of formula (II):
RI (I)
R'OH (II)
to give a labeled ester of formula (III): 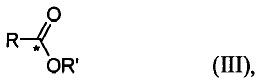 wherein R is linear or cyclic alkyl or substituted alkyl, aryl or substituted aryl, and may contain fluoro, ester and carboxyl groups, which are separated by at least one carbon atom from the carbon atom bearing the iodide atom; R' is H, linear or cyclic alkyl or substituted alkyl, and may contain fluoro, ester and carboxyl groups; and wherein the photosensitizer is any organic or inorganic compound that is capable of absorbing photo irradiation and transferring the gained energy to the reactants.

7. A method of claim 6, wherein the photosensitizers are aliphatic or aromatic ketones or aryl amines.

8. A method of claim 6, wherein the photosensitizers are acetone, benzophenone, xanthone or triphenylamine.

9. A method of claim 6, wherein the alcohol is a primary or secondary.

## Patentansprüche

1. Verfahren zur markierenden Synthese, bestehend aus:
(a) Bereitstellen einer UV-Reaktoranordnung, umfassend eine Hochdruckreaktionskammer, eine UV-Spot-Lichtquelle mit einem Lichtleiter, wobei der Lichtleiter verwendet wird, um Lichtbestrahlung eines Reaktionsgemischs durch ein Fenster in der Reaktionskammer bereitzustellen,
(b) Lösen eines Alkohols und eines Photosensibilisators,
(c) Zugeben eines Alkyl- oder Aryliodids zur Lösung aus Schritt (b), damit sich ein zu markierendes Reagensvolumen ergibt,
(d) Einlassen eines [¹¹C]-Kohlenmonoxid-angereicherten Gasgemischs in die Reaktionskammer der UV-Reaktoranordnung via den Gaseinlass,
(e) Einlassen des Reagensvolumens unter hohem Druck in die Reaktionskammer via den Flüssigkeitseinlass,
(f) Anschalten der UV-Spot-Lichtquelle und Warten eine vorgegebene Zeit lang, während die markierende Synthese erfolgt, und
(g) Sammeln des markierten Esters aus der Reaktionskammer.

2. Verfahren nach Anspruch 1, wobei das [¹¹C]-Kohlenmonoxid-angereicherte Gasgemisch durch ein Verfahren hergestellt wird, das umfasst:
(a) Bereitstellen von [¹¹C]-Kohlendioxid in einem geeigneten Trägergas,
(b) Umwandeln von [¹¹C]-Kohlendioxid zu [¹¹C]-Kohlenmonoxid durch Einlassen des Gasgemischs in eine Reaktorvorrichtung,
(c) Fangen von [¹¹C]-Kohlenmonoxid in einer Kohlenmonoxidfallenvorrichtung, wobei [¹¹C]-Kohlenmonoxid, aber nicht das Trägergas, gefangen wird, und
(d) Freisetzen des gefangenen [¹¹C]-Kohlenmonoxids aus der Fallenvorrichtung in einem gut definierten Mikro-Plug, wodurch ein Volumen von [¹¹C]-Kohlenmonoxid-angereichertem Gasgemisch erzielt wird.

3. Verfahren nach Anspruch 1, wobei der Schritt des Einlassens des Reagens unter Anwendung eines Drucks, der 80-mal höher ist als der Druck vor dem Einlassen, durchgeführt wird, um ein Pseudoeinphasensystem aufrechtzuerhalten, wobei "Pseudoeinphasensystem" ein geschlossenes Volumen mit einem geringen Verhältnis von Oberflächenbereich zu Volumen bedeutet, das mehr als 96% Flüssigkeit und weniger als 4% Gas bei Drücken über 200 bar enthält.

4. Verfahren nach Anspruch 1, wobei der Schritt, eine vorgegebene Zeit lang zu warten, das Rühren in der Reaktionskammer umfasst, um die markierende Synthese zu verbessern.

5. Verfahren nach Anspruch 4, wobei der Schritt, eine vorgegebene Zeit lang zu warten, weiterhin das Regulieren der Temperatur der Reaktionskammer so umfasst, dass die markierende Synthese verbessert wird.

6. Verfahren nach Anspruch 1, wobei das Alkyl- oder Aryliodid eine Verbindung der Formel (1) ist und der Alkohol ein Alkohol der Formel (II) ist:
RI (I)
R'OH (II),
damit sich ein markierter Ester der Formel (III) ergibt: 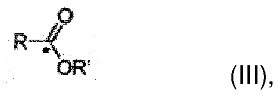 worin R für lineares oder zyklisches Alkyl oder substituiertes Alkyl, Aryl oder substituiertes Aryl steht, und Fluor-, Ester- und Carboxygruppen enthalten kann, die durch zumindest ein Kohlenstoffatom von dem Kohlenstoffatom getrennt sind, das das lodidatom trägt; R' für H, lineares oder zyklisches Alkyl oder substituiertes Alkyl steht, und Fluor-, Ester- und Carboxygruppen enthalten kann; und wobei der Photosensibilisator eine beliebige organische oder anorganische Verbindung ist, die in der Lage zum Absorbieren von Lichtbestrahlung und zum Übertragen der gewonnenen Energie auf die Reaktanten ist.

7. Verfahren nach Anspruch 6, wobei die Photosensibilisatoren aliphatische oder aromatische Ketone oder Arylamine sind.

8. Verfahren nach Anspruch 6, wobei die Photosensibilisatoren in Aceton, Benzophenon, Xanthon oder Triphenylamin bestehen.

9. Verfahren nach Anspruch 6, wobei der Alkohol ein primärer oder sekundärer ist.

## Revendications

1. Procédé de synthèse de marquage, consistant à :
(a) préparer un ensemble formant réacteur à UV comprenant une chambre de réaction à haute pression, une source de lumière UV ponctuelle avec un guide de lumière, dans lequel le guide de lumière est utilisé afin d'assurer l'irradiation lumineuse d'un mélange de réaction à travers une fenêtre dans la chambre de réaction,
(b) dissoudre un alcool et un agent de photosensibilisation,
(c) ajouter un iodure d'alkyle ou d'aryle à la solution de l'étape (b) afin d'obtenir un volume de réactif à marquer,
(d) introduire un mélange de gaz enrichi en monoxyde de carbone marqué [¹¹C] dans la chambre de réaction de l'ensemble formant réacteur à UV par l'intermédiaire de l'entrée de gaz,
(e) introduire à haute pression ledit volume de réactif dans la chambre de réaction par l'intermédiaire de l'entrée de liquide,
(f) activer la source de lumière UV ponctuelle et attendre pendant une durée prédéterminée que la synthèse de marquage se produise, et
(g) collecter l'ester marqué dans la chambre de réaction.

2. Procédé selon la revendication 1, dans lequel le mélange de gaz enrichi en monoxyde de carbone marqué [¹¹C] est produit par un procédé comprenant :
(a) l'introduction de dioxyde de carbone marqué [¹¹C] dans un gaz porteur approprié,
(b) la transformation du dioxyde de carbone marqué [¹¹C] en monoxyde de carbone marqué [¹¹C] en introduisant ledit mélange de gaz dans un dispositif formant réacteur,
(c) le piégeage du monoxyde de carbone marqué [¹¹C] dans un dispositif de piégeage de monoxyde de carbone, dans lequel le monoxyde de carbone marqué [¹¹C] est piégé mais pas ledit gaz porteur , et
(d) la libération dudit monoxyde de carbone marqué [¹¹C] piégé dudit dispositif de piégeage dans une micro-capsule bien définie, de telle sorte qu'un volume de mélange de gaz enrichi en monoxyde de carbone marqué [¹¹C] est obtenu.

3. Procédé selon la revendication 1, dans lequel l'étape d'introduction du réactif est mise en oeuvre en utilisant une pression qui est de 80 fois supérieure à la pression avant l'introduction, afin de conserver un système à une pseudo-phase dans lequel le "système à une pseudo-phase" signifie un volume fermé avec un faible rapport de surface efficace sur volume contenant plus de 96% de liquide et moins de 4% de gaz à des pressions excédant 200 bars.

4. Procédé selon la revendication 1, dans lequel l'étape d'attente d'une durée prédéterminée comprend l'agitation dans la chambre de réaction de manière à améliorer la synthèse de marquage.

5. Procédé selon la revendication 4, dans lequel l'étape d'attente d'une durée prédéterminée comprend, en outre, l'ajustement de la température de la chambre de réaction de telle sorte que la synthèse de marquage soit améliorée.

6. Procédé selon la revendication 1 dans lequel ledit iodure d'alkyle ou d'aryle est un composé de formule (I) et ledit alcool est un alcool de formule (II) :
RI (I)
R'OH (II)
afin d'obtenir un ester marqué de formule (III): 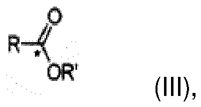 dans laquelle R représente un alkyle ou alkyle substitué, un aryle ou aryle substitué linéaire ou cyclique, et peut contenir des groupes fluoro, ester et carboxyle, qui sont séparés par au moins un atome de carbone par rapport à l'atome de carbone supportant l'atome d'iodure ; R' représente H, un alkyle ou alkyle substitué linéaire ou cyclique, et peut contenir des groupes fluoro, ester et carboxyle ; et
dans lequel l'agent de photosensibilisation est un composé organique ou inorganique quelconque qui peut absorber l'irradiation lumineuse et transférer l'énergie acquise aux réactifs.

7. Procédé selon la revendication 6, dans lequel les agents de photosensibilisation sont des cétones aliphatiques ou aromatiques ou des amines d'aryle.

8. Procédé selon la revendication 6, dans lequel les agents de photosensibilisation sont l'acétone, la benzophénone, la xanthone ou la triphénylamine.

9. Procédé selon la revendication 6, dans lequel l'alcool est un alcool primaire ou secondaire.
